# EUROPÄISCHE PATENTSCHRIFT

(11) **EP 1 052 253 B1**
(45) Veröffentlichungstag und Bekanntmachung des Hinweises auf die Patenterteilung: **08.04.2009**
(21) Anmeldenummer: 00108148.8
(22) Anmeldetag: 13.04.2000
(51) Int. Cl.: C07D 213/80, C07D 213/79

(54) **Verfahren zur Herstellung von 2,3-Pyridindicarbonsäuren**
Process for the preparation of 2,3-pyridinedicarboxylic acids
Procédé pour la préparation d'acides pyridine-2,3-dicarboxyliques

(30) Priorität: 10.05.1999 AT 83299
(43) Veröffentlichungstag der Anmeldung: 15.11.2000
(73) Patentinhaber: DSM Fine Chemicals Austria Nfg GmbH & Co KG, 4021 Linz (AT)
(72) Erfinder: Giselbrecht, Karlheinz, 4061 Pasching (AT); Perndorfer, Eduard, 4060 Traun (AT); Reiter, Klaus, 4020 Linz (AT)
(74) Vertreter: Habets, Winand

(56) Entgegenhaltungen:
- US-A- 2 964 529
- O'MURCHU C: "Ozonolysis of Quinolines: A Versatile Synthesis of Polyfunctional Pyridines" SYNTHESIS, Nr. 11, 1. November 1989 (1989-11-01), Seiten 880-882, XP002037527 ISSN: 0039-7881

## Beschreibung

2,3-Pyridindicarbonsäuren (PDCA) stellen einen wichtigen Rohstoff zur Synthese von Pharma- und Agrochemikalien dar.
Aus der Literatur sind bereits verschiedene Verfahren zur Herstellung von PDCA bekannt. Sie beruhen teils auf der Oxidation von Chinolin und teils auf der Oxidation von am aromatischen Kern aktivierend substituierten Chinolinderivaten.
Die zuerst von Hoogewerff und van Dorp in Chem. Ber., Seite 425f, (1883) beschriebene Methode der Oxidation von Chinolin aus Steinkohlenteer mit Kaliumpermanganat im alkalischen Milieu erbringt jedoch nur sehr geringe Ausbeuten an PDCA neben einer großen Menge an Nebenprodukten.
Die weiteren Verfahren zur Oxidation von Chinolin leiten sich im wesentlichen von der von Stix und Bulgatsch in Chem. Ber. Seite 11f, (1932) beschriebenen Methode der Oxidation mit Wasserstoffperoxid in Anwesenheit eines Kupfersalzes ab. Da diese Reaktion recht schwierig zu handhaben ist, wurden bereits mehrere Verbesserungen vorgeschlagen, die eine bessere Steuerung der Reaktion und eine leichte Erhöhung der Ausbeute bedingen.
Beispiele dafür sind EP-A-0 024 197 oder EP-A-0 034 943. Bei all diesen Varianten entsteht jedoch immer zuerst ein Kupfersalz von PDCA, aus welchem die freie Säure mittels eines Sulfids freigesetzt werden muss. Ein weiterer Nachteil ist, dass eine vollständige Abtrennung der Kupferionen äußerst schwierig ist, sodass so hergestellte PDCA immer Spuren von Kupfer enthält.
DE-A-31 50 005 beschreibt ein weiteres Oxidationsverfahren, bei dem Chinolinderivate mit Chlorationen oxidiert werden, wobei Vanadyl(V)-Kationen als Katalysator dienen. Dieses Verfahren eignet sich aber nur für solche Chinolinderivate, in denen am Benzolkern mindestens ein Wasserstoffatom durch eine aktivierende Gruppe substituiert ist, während das leicht zugängliche und preiswertere unsubstituierte Chinolin nicht mit diesem Verfahren oxidiert werden kann.
Weitere Oxidationsverfahren, wie etwa in DE-A-33 45 223 beschrieben liefern das gewünschte Endprodukt PDCA nur in geringen Ausbeuten von etwa 52%.

Eine andere Methode, die Ozonolyse von Chinolin oder Chinolinderivaten, zur Herstellung von Pyridindicarbonsäuren ist beispielsweise in US 2,964,529 beschrieben. Gemäß US 2,964,529 werden Benzazine, aus der Gruppe bestehend aus Chinolin, Isochinolin und substituierten Chinolinen und Isochinolinen, im ersten Schritt mit Ozon bevorzugt bei Temperaturen von 25 bis 65°C in Gegenwart von mindestens einem Mol Mineralsäure, bevorzugt HNO₃, pro Mol Benzazin, und anschließend bei erhöhter Temperatur mit einem Oxidationsmittel umgesetzt wird. Wie Erfahrungswerte, bzw. die Farbe der Endprodukte zeigten, ist die Reinheit der erhaltenen Pyridindicarbonsäuren jedoch nicht zufriedenstellend. Dies tritt insbesondere dann auf, wenn von Chinolin, das direkt aus der Steinkohleteerdestillation kommt und bis zu 5% Isochinolin enthält, ausgegangen wird. Isochinolinverunreinigungen sind bei der Herstellung von 2,3-Pyridindicarbonsäuren sehr störend, da sie die entsprechend schlechter lösliche 3,4-Pyridindicarbonsäure bilden.
Gemäß O'Murchu, Synthesis (1989) S. 880-882 werden Pyridincarbonsäuren aus den entsprechenden substituierten Chinolinen im ersten Schritt durch Ozonolyse hergestellt, wobei das Chinolin-Ausgangsprodukt, welches eine basische Funktion hat, durch Zusatz von Schwefelsäure als Sulfat in einem Gemisch aus 15 Vol.% Wasser und 85 Vol.% Essigsäure gelöst und die Ozonolyse in dieser Lösung bei 35°C durchgeführt wird. Die Ozonolyse dauert dabei zwischen 20 und 30 Stunden. Anschließend wird das Reaktionsgemisch mit Wasserstoffperoxid weiter oxidiert. Die Reaktionsprodukte werden dabei in Ausbeuten von 20 bis max. 84% erhalten.

Aufgabe der Erfindung war es demnach ein verbessertes Verfahren zur Herstellung von 2,3-Pyridindicarbonsäuren zu finden, bei welchem, auch bei Anwesenheit von größeren Mengen an Isochinolin im Ausgangsprodukt, die gewünschten 2,3-Pyridindicarbonsäuren in hoher Ausbeute und hoher Reinheit erhalten werden.

Gegenstand der Erfindung ist daher ein Verfahren zur Herstellung von reinen 2,3-Pyridindicarbonsäuren der Formel I 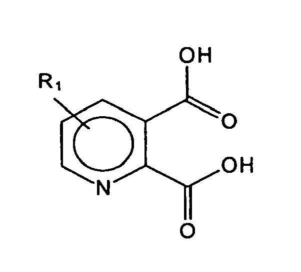 die in Position 4 und/oder 5 und/oder 6 durch R₁ substituiert sind, wobei R₁ Wasserstoff, C₁-C₄Alkyl, C₁-C₄Alkoxy, C₁-C₄Alkoxy-C₁-C₄Alkyl, Halogen, Hydroxyl oder Nitro bedeutet, durch Ozonolyse in wässriger, mineralsaurer Lösung und anschließender Oxidation in Gegenwart eines Oxidationsmittels, das dadurch gekennzeichnet ist, dass Chinoline der Formel 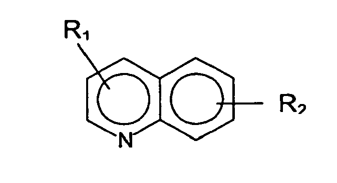 in der R₁ wie oben definiert ist, und die in Position 6 und/oder 7 durch R₂ substituiert sind, wobei R₂ Wasserstoff, C₁-C₄Alkyl, C₁-C₄Alkoxy, C₁-C₄Alkoxy-C₁-C₄Alkyl, Halogen, Hydroxyl, Nitro oder Amino bedeutet, im ersten Schritt in schwefel- oder salpetersaurer, wässriger Lösung mit Ozon im Verhältnis von 1 : 2 bis 1 : 3 bei Temperaturen von 0 bis +10°C und anschließend die so erhaltene Peroxidlösung bei Temperaturen von +0 bis +100°C in Gegenwart von 0,5-4,0 Mol Oxidationsmittel pro mol gebildetes Ozonolyseprodukt umgesetzt werden, worauf der pH-Wert der Reaktionslösung auf 0,2 bis 3 gestellt, auf 0 bis 30°C abgekühlt und die ausgefallene Pyridindicarbonsäure isoliert wird.

Bei dem erfindungsgemäßen Verfahren werden 2,3-Pyridindicarbonsäuren der Formel I hergestellt. Dabei wird von Chinolinen der Formel II ausgegangen, die durch die Reste R₁ und R₂ substituiert sind. R₁ befindet sich in der Formel II in Position 2 und/oder 3 und/oder 4 und bedeutet Wasserstoff, C₁-C₄Alkyl, C₁-C₄Alkoxy, C₁-C₄Alkoxy-C₁-C₄Alkyl, Halogen, Hydroxyl, oder Nitro. Bevorzugt ist nur eine der Positionen 2, 3 oder 4 durch einen Rest R₁, der nicht Wasserstoff bedeutet, substituiert. Besonders bevorzugt bedeutet R₁ in Position 2und 4 Wasserstoff und in Position 3 Methyl, Ethyl oder Methoxymethyl. Weiters weisen die Chinoline der Formel II in Position 6 und/oder 7 den Substituenten R₂ auf. R₂ bedeutet eine unter den Reaktionsbedingungen inerte Gruppe wie Wasserstoff, C₁-C₄Alkyl, C₁-C₄Alkoxy, C₁-C₄Alkoxy-C₁-C₄Alkyl , Halogen, Hydroxyl, Nitro oder Amino.
Bevorzugt bedeutet R₂ Wasserstoff.
Besonders bevorzugt wird demnach ein unsubstituiertes oder ein in Position 3 durch Methyl, Ethyl oder Methoxymethyl substituiertes Chinolin eingesetzt. Erfindungsgemäß kann sowohl reines Chinolin als auch Chinolin, welches direkt, d.h. ohne Reinigungsschritt, aus der Steinkohleteerdestillation kommt und bis zu 5% Isochinolin enthält, eingesetzt werden. Die Chinoline der Formel II werden erfindungsgemäß in wässriger, schwefel- oder salpetersaurer Lösung zu den entsprechenden 2,3-Pyridindicarbonsäuren umgesetzt. Bevorzugt wird Schwefelsäure eingesetzt. Dem Reaktionsgemisch wird dabei soviel Säure zugesetzt, dass der pH-Wert der Reaktionslösung zwischen 0,1 und 4, bevorzugt zwischen 0,3 und 1,5 und besonders bevorzugt zwischen 0,4 und 1 liegt. Die Konzentration an Edukt liegt bevorzugt zwischen 2 und 30 Gew%, besonders bevorzugt zwischen 2,5 und 10 Gew%.
Die wässrige, schwefel- oder salpetersaure Chinolinlösung wird mit Ozon in einem Chinolin:Ozon-Verhältnis von 1:2 bis 1:3, bevorzugt bis 1:2,5; besonders bevorzugt bis 1:2,3 versetzt. Dazu wird die Reaktionslösung bevorzugt in einer Umlaufapparatur oder in einer geeigneten Batch-Apparatur mit einem ozonführenden O₂-Strom begast, bis die entsprechende Ozonmenge aufgenommen wurde. Das Ende der Reaktion ist dabei meist bei dem theoretischen Ozonverbrauch erreicht. Bevorzugt wird das Ende der Reaktion durch eine geeignete Inprozeßkontrolle auf das Abreagieren des Chinolins festgestellt. Die Temperatur bei der Ozonolyse beträgt 0 bis 10°C und bevorzugt 2 bis 5°C.

Gegebenenfalls kann der Zusatz eines gegen Ozon beständigen Antischaummittels zur Ozonolyselösung von Vorteil sein. Bevorzugt werden dann Antischaummittel auf Siliconbasis eingesetzt, wobei die Menge an zugesetztem Antischaummittel abhängig ist vom Ausmaß der Neigung zum Schäumen und bevorzugt etwa 0,01 bis 0,2 Vol%, besonders bevorzugt 0,05 bis 0,15 Vol%, bezogen auf die Gesamtmenge an Ozonolyselösung, beträgt.

Anschließend an den Ozonolyseschritt erfolgt die Oxidation der erhaltenen Peroxidlösung. Dazu wird die Peroxidlösung mit einem geeigneten Oxidationsmittel versetzt. Als Oxidationsmittel kommen Wasserstoffperoxid, Hypochlorid, Persäuren, Peroxodisulfat, Perborate, Kaliumpermanganat u.s.w. in Frage. Die Oxidation kann auch katalytisch mit Sauerstoff in Anwesenheit von Übergangsmetallkatalysatoren erfolgen. Bevorzugt wird Wasserstoffperoxid in Form einer 3 bis 70%igen Lösung, besonders bevorzugt als 20 bis 50%ige Lösung verwendet. Die Menge an Oxidationsmittel liegt dabei zwischen 0,5 und 4,0 Mol pro Mol gebildetes Ozonolyseprodukt beziehungsweise zwischen 0,5 und 1,5 Moläquivalenten (mequ), bevorzugt zwischen 0,7 und 1,2, besonders bevorzugt zwischen 0,8 und 1 mequ an Wasserstoffperoxid (bzw. an Oxidationsmittel), bezogen auf das eingesetzte Chinolin. Die Temperatur während der Oxidation beträgt 0 bis +100°C, bevorzugt zwischen 10 und 70°C, besonders bevorzugt zwischen 15 und 50°C.
Nach beendeter Reaktion, d.h. nach etwa 1 bis 24 Stunden wird das erhaltene Reaktionsgemisch alkalisiert, bis ein pH-Wert zwischen 0,2 und 3, bevorzugt zwischen 0,7 und 2 und besonders bevorzugt zwischen 0,9 und 1,1 erreicht ist. Zur Alkalisierung werden übliche basische Zusätze, wie etwa NaOH, KOH u.s.w. verwendet. Bevorzugt wird NaOH oder KOH, besonders bevorzugt 40 bis 50%ige NaOH eingesetzt. Gleichzeitig oder anschließend wird das Reaktionsgemisch auf 0 bis 30°C, bevorzugt auf 4 bis 10°C abgekühlt, wodurch die entsprechende 2,3-Pyridindicarbonsäure auskristallisiert.
Zur Isolierung des gewünschten Endproduktes wird der angefallene Feststoff abgesaugt, abzentrifugiert oder abfiltriert, gewaschen, bevorzugt mit Wasser und Methanol und anschließend getrocknet. Durch Aufarbeiten der Mutterlauge, z.B. durch Einengen kann die Ausbeute an gewünschtem Endprodukt um bis zu 10% gesteigert werden.

Das erfindungsgemäße Verfahren eignet sich somit zur Herstellung von 2,3-Pyridindicarbonsäuren der Formel I, insbesondere zur Herstellung von unsubstituierter oder in Position 5 durch Methyl, Ethyl oder Methoxymethyl substituierter 2,3-Pyridindicarbonsäure in hohen Ausbeuten von über 80% mit einem Gehalt von über 99,8%.
Dies ist insbesondere bei der Verwendung von Chinolin, das direkt aus der Steinkohleteerdestillation kommt und bis zu 5% an Isochinolin als Verunreinigung enthält, von großem Vorteil. Im Vergleich zum Stand der Technik werden mit dem erfindungsgemäßen Verfahren gerade bei diesem verunreinigtem Ausgangsprodukt wesentlich höhere Reinheiten des Endproduktes erzielt. Dies ist bei der Weiterverarbeitung der 2,3-Pyridindicarbonsäuren zu den entsprechenden Pharma- und Agrarchemikalien von großer Bedeutung.

### Beispiel 1:

140g (1,08 mol) Chinolin 97,5%, Fa. Sumitomo, enthaltend1,5% Isochinolin, wurden in 1770g destilliertem Wasser und 212,6g 98%iger Schwefelsäure (2,16 mol) gelöst, wodurch eine etwa 7%ige Lösung mit einem pH-Wert von 0,3 erhalten wurde. Diese Lösung wurde bei 6°C mit einem ozonführenden O₂-Strom in einer Kreislaufozonisierungsanlage begast. Die Dauer der Ozonolysereaktion betrug 8 h und 40 min, wobei 118g (2,46mol) Ozon verbraucht wurden. Drei Tropfen Antifoam (Antifoam SRE, Fa. Wacker) mit 50ml Wasser verdünnt wurden nach Bedarf zugegeben. Insgesamt wurden 17 ml der Entschäumerlösung benötigt.
Nach beendeter Ozonolyse wurden 2094g Peroxidlösung erhalten, die in einem auf 20°C vorgekühlten Reaktionsgefäß unter Rühren mit 216g (1,86mol) Wasserstoffperoxid (30%ig) versetzt wurde. Die Temperatur stieg dabei auf 24°C an. Das Reaktionsgemisch wurde über Nacht gerührt und anschließend bei einer Temperatur zwischen 20 und 30°C mit 305g 40%iger NaOH versetzt, wodurch der pH-Wert von 0,3 auf 1,0 gestellt wurde. Danach wurde auf 8°C gekühlt, sodass 2,3-Pyridindicarbonsäure auskristallisierte.
Der angefallene Feststoff wurde abgesaugt mit Wasser und Metahnol gewaschen (2 mal mit je 100ml) und getrocknet.
Ausbeute: 125g (71,14%) mit einem Gehalt von 99,7% (GC+HPLC)
Nach Aufarbeitung der Mutterlauge durch Einengen wurden weitere 5% an 2,3-Pyridindicarbonsäure erhalten.
Die Analyse ergab, dass das Endprodukt keine Verunreinigung durch 3,4-Pridincarbonsäure enthielt.

## Patentansprüche

1. Verfahren zur Herstellung von reinen 2,3-Pyridindicarbonsäuren der Formel I 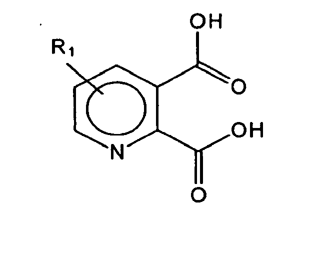 die in Position 4 und/oder 5 und/oder 6 durch R₁ substituiert sind, wobei R₁ Wasserstoff, C₁-C₄Alkyl, C₁-C₄Alkoxy, C₁-C₄Alkoxy-C₁-C₄Alkyl, Halogen, Hydroxyl oder Nitro bedeutet, durch Ozonolyse in wässriger, mineralsaurer Lösung und anschließender Oxidation in Gegenwart eines Oxidationsmittels, **dadurch gekennzeichnet, dass** Chinoline der Formel II 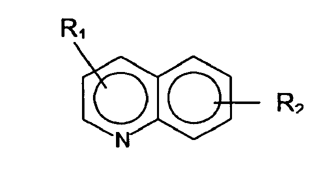 in der R₁ wie oben definiert ist, und die in Position 6 und/oder 7 durch R₂ substituiert sind, wobei R₂ Wasserstoff, C₁-C₄Alkyl, C₁-C₄Alkoxy, C₁-C₄Alkoxy-C₁-C₄Alkyl, Halogen, Hydroxyl, Nitro oder Amino bedeutet, im ersten Schritt in schwefel- oder salpetersaurer, wässriger Lösung mit Ozon im Verhältnis von 1 : 2 bis 1 : 3 bei Temperaturen von 0 bis +10°C und anschließend die so erhaltene Peroxidlösung bei Temperaturen von +0 bis +100°C in Gegenwart von 0,5-4,0 Mol Oxidationsmittel pro mol gebildetes Ozonolyseprodukt umgesetzt werden, worauf der pH-Wert der Reaktionslösung auf 0,2 bis 3 gestellt, auf 0 bis 30°C abgekühlt und die ausgefallene Pyridindicarbonsäure isoliert wird.

2. Verfahren nach Anspruch 1, **dadurch gekennzeichnet, dass** als Chinolin der Formel II ein reines Chinolin oder ein mit bis zu 5% Isochinolin verunreinigtes Chinolin eingesetzt wird.

3. Verfahren nach Anspruch 1, **dadurch gekennzeichnet, dass** Chinoline der Formel II, in der R₁ in Position 2 und 4 Wasserstoff und in Position 3 Methyl, Ethyl oder Methoxymethyl und R₂ Wasserstoff bedeutet, eingesetzt werden.

4. Verfahren nach Anspruch 1, **dadurch gekennzeichnet, dass** im ersten Schritt soviel Säure zugegeben wird, dass ein pH-Wert zwischen 0,3 und 1,5 eingestellt wird.

5. Verfahren nach Anspruch 1, **dadurch gekennzeichnet, dass** 0,5 bis 1,5 Moläquivalente an Oxidationsmittel, bezogen auf das eingesetzte Chinolin, eingesetzt werden.

6. Verfahren nach Anspruch 1, **dadurch gekennzeichnet, dass** die Temperatur bei der Oxidation zwischen 15 und 50°C liegt.

7. Verfahren nach Anspruch 1, **dadurch gekennzeichnet, dass** nach der Oxidation der pH-Wert der Reaktionslösung auf einen Wert zwischen 0,9 und 1,1 gestellt wird.

8. Verfahren nach Anspruch 1, **dadurch gekennzeichnet, dass** zur Ausfällung der Pyridindicarbonsäure die Reaktionslösung auf eine Temperatur zwischen 4 und 10°C abgekühlt wird.

## Claims

1. Process for the preparation of pure 2,3-pyridinedicarboxylic acids of the formula I 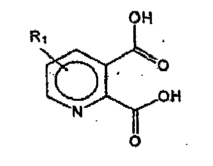 which are substituted in position 4 and/or 5 and/or 6 by R₁, where R₁ is hydrogen, C₁-C₄ alkyl, C₁-C₄ alkoxy, C₁-C₄ alkoxy-C₁-C₄ alkyl, halogen, hydroxyl or nitro, by ozonolysis in aqueous, mineral acid solution and subsequent oxidation in the presence of an oxidizing agent, **characterized in that** quinolines of the formula II 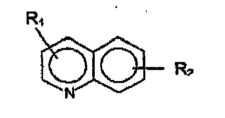 in which R₁ is as defined above, and which are substituted in position 6 and/or 7 by R₂, where R₂ is hydrogen, C₁-C₄ alkyl, C₁-C₄ alkoxy, C₁-C₄ alkoxy-C₁-C₄ alkyl, halogen, hydroxy, nitro or amino, are reacted in the first step in aqueous sulphuric acid or nitric acid solution with ozone in the ratio from 1:2 to 1:3 at temperatures from 0 to +10°C, and then the resulting peroxide solution is reacted at temperatures of from +0 to +100°C in the presence of 0.5-4.0 mol of oxidizing agent per mole of ozonolysis product formed, then the pH of the reaction solution is adjusted to 0.2 to 3, the mixture is cooled to 0 to 30°C, and the precipitated pyridinedicarboxylic acid is isolated.

2. Process according to claim 1, **characterized in that** the quinoline of the formula II used is a pure quinoline or a quinoline contaminated with up to 5% of isoquinoline.

3. Process according to claim 1, **characterized in that** quinolines of the formula II in which R₁ in position 2 and 4 is hydrogen, and in position 3 is methyl, ethyl or methoxymethyl, and R₂ is hydrogen are used.

4. Process according to claim 1, **characterized in that,** in the first step, enough acid is added to adjust the pH to between 0.3 and 1.5.

5. Process according to claim 1, **characterized in that** 0.5 to 1.5 mol equivalents of oxidizing agent, based on the quinoline used, are used.

6. Process according to claim 1, **characterized in that** the temperature during the oxidation is between 15 and 50°C.

7. Process according to claim 1, **characterized in that,** hollowing the oxidation, the pH of the reaction solution is adjusted to a value between 0.9 and 1.1.

8. Process according to claim 1, **characterized in that,** to precipitate out the pyridinedicarboxylic acid, the reaction solution is cooled to a temperature between 4 and 10°C.

## Revendications

1. Procédé pour la préparation d'acides 2,3-pyridine-dicarboxyliques purs de formule I 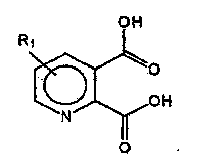 qui sont substitués en position 4 et/ou 5 et/ou 6 par R₁, R₁ représentant un atome d'hydrogène ou d'halogène ou un groupe alkyle en C₁-C₄, alcoxy en C₁-C₄, alcoxy(C₁-C₄)-alkyle(C₁-C₄), hydroxy ou nitro, par ozonolyse en solution aqueuse, acidifiée par un acide minéral, et oxydation subséquente en présence d'un oxydant, **caractérisé en ce qu'**on fait réagir des quinoléines de formule II 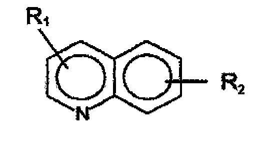 dans laquelle R₁ est tel que défini ci-dessus, et qui sont substituées en position 6 et/ou 7 par R₂, R₂ représentant un atome d'hydrogène ou d'halogène ou un groupe alkyle en C₁-C₄, alcoxy en C₁-C₄, alcoxy(C₁-C₄)-alkyle(C₁-C₄), hydroxy, nitro ou amino, dans la première étape en solution aqueuse, acidifiée par de l'acide nitrique ou sulfurique, avec de l'ozone en un rapport de 1:2 à 1:3, à des températures de 0 à +10 °C, et ensuite on fait réagir la solution de peroxyde obtenue, à des températures de 0 à +100 °C en présence de 0,5-4,0 moles d'oxydant par mole de produit d'ozonolyse formée, à la suite de quoi on ajuste le pH de la solution réactionnelle à 0,2-3, on la refroidit jusqu'à 0-30 °C et on isole l'acide pyridinedicarboxylique précipité.

2. Procédé selon la revendication 1, **caractérisé en ce qu'**on utilise en tant que quinoléine de formule II une quinoléine pure ou une quinoléine contaminée avec jusqu'à 5 % d'isoquinoléine.

3. Procédé selon la revendication 1, **caractérisé en ce qu'**on utilise des quinoléines de formule II dans lesquelles R₁ représente en position 2 à 4 un atome d'hydrogène et en position 3 un groupe méthyle, éthyle ou méthoxyméthyle et R₂ représente un atome d'hydrogène.

4. Procédé selon la revendication 1, **caractérisé en ce que** dans la première étape on ajoute suffisamment d'acide pour ajuster un pH compris entre 0,3 et 1,5.

5. Procédé selon la revendication 1, **caractérisé en ce qu'**on utilise de 0,5 à 1,5 équivalent molaire d'oxydant, par rapport à la quinoléine utilisée.

6. Procédé selon la revendication 1, **caractérisé en ce que** lors de l'oxydation la température est comprise entre 15 et 50 °C.

7. Procédé selon la revendication 1, **caractérisé en ce qu'**après l'oxydation on ajuste le pH de la solution réactionnelle à une valeur comprise entre 0,9 et 1,1.

8. Procédé selon la revendication 1, **caractérisé en ce que** pour la précipitation de l'acide pyridinedicarboxylique on refroidit la solution réactionnelle jusqu'à une température comprise entre 4 et 10 °C.
